(19) Europäisches Patentamt
European Patent Office
Office européen des brevets

(11) Veröffentlichungsnummer: **0 047 459**
**B1**

(12) # EUROPÄISCHE PATENTSCHRIFT

(45) Veröffentlichungstag der Patentschrift: **21.11.84**

(51) Int. Cl.³: **G 01 N 33/58**, G 01 N 33/94

(21) Anmeldenummer: **81106776.8**

(22) Anmeldetag: **29.08.81**

(54) **Fluoreszierendes Reagenz und Immunofluoreszenz-Bestimmungsmethode.**

(30) Priorität: **08.09.80 US 185235**

(43) Veröffentlichungstag der Anmeldung: **17.03.82 Patentblatt 82/11**

(45) Bekanntmachung des Hinweises auf die Patenterteilung: **21.11.84 Patentblatt 84/47**

(84) Benannte Vertragsstaaten: **AT BE CH DE FR GB IT LI LU NL SE**

(56) Entgegenhaltungen:
**EP-A-0 003 583**
**DE-A-2 430 356**
**DE-A-2 716 276**
**DE-A-2 727 208**
**GB-A-2 039 484**
**GB-A-2 039 485**
**US-A-4 169 137**

(73) Patentinhaber: **INTERNATIONAL DIAGNOSTIC TECHNOLOGY, INC., 2551 Walsh Avenue, Santa Clara, CA 95050 (US)**

(72) Erfinder: **Tsay, Yuh-Geng, 3006 Postwood Drive, San Jose California 95132 (US)**
Erfinder: **Chen, Janet H., 2517 Greengate Drive, San Jose California 95132 (US)**
Erfinder: **Palmer, Richard J., 1623 Santa Clara Street, Santa Clara California 95050 (US)**

(74) Vertreter: **Laudien, Dieter, Boehringer Ingelheim International GmbH ZA Patente Postfach 200, D-6507 Ingelheim am Rhein- (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 047 459 B1

BUNDESDRUCKEREI BERLIN

**Beschreibung**

Die Erfindung betrifft ein verbessertes fluoreszierendes Reagenz und eine Immunofluoreszenz-Bestimmungsmethode.

Bei den bekannten Immunofluoreszenz-Bestimmungsmethoden an festen Oberflächen wird der zu analysierende Körper, beispielsweise ein Hapten, mit einem fluoreszierenden Bestandteil markiert und in Konkurrenz mit einem unmarkierten Hapten durch Adsorption oder in anderer Weise an einer Oberfläche fixiert (Tsay et al., »Quantitation of Serum Gentamicin Concentration by a Solid Phase Immunofluorescence Method«, Clin. Chem., Vol. 26 (1980)). Beim Eintauchen dieser Oberfläche, an die eine bestimmte Menge eines Antikörpers gebunden ist, in eine Serumprobe, wird eine der Bindungsmöglichkeit des Antikörpers entsprechende begrenzte Menge an markiertem und unmarkiertem Hapten immunologisch gebunden. Nach dem Herausnehmen der Oberfläche aus dem Serum wird mit Hilfe eines Präzisionsfluorometers die Hapten-Antigen-Konzentration im Serum bzw. Blut gemessen. Als Präzisionsfluorometer eignet sich das FIAX®-System 100, und zwar sowohl für qualitative als auch für quantitative Messungen.

Die Immunofluoreszenz-Bestimmung an einer festen Oberfläche ist eine schnelle, einfache und vergleichsweise genaue Methode zur quantitativen Bestimmung des zu analysierenden Körpers. Sie besitzt jedoch wenigstens drei schwerwiegende Nachteile, nämlich nichtspezifische Bindungen an der Oberfläche, verminderte Antigenität und nicht spezifische Bindung mit anderen Serumbestandteilen und die hierdurch bedingte Fluoreszenzlöschung. Aufgabe der vorliegenden Erfindung ist die Überwindung dieser Nachteile.

Es ist anzunehmen, daß die hohe Anzahl der nichtspezifischen Bindungen des markierten Haptens an der Oberfläche im wesentlichen auf hydrophobe Wechselwirkungen zwischen der festen Oberfläche und dem fluoreszenzmarkierten Hapten-Komplex zurückzuführen ist. Wenn beispielsweise die fluoreszierende Verbindung oder die Markierung Fluoresceinisothiocyanat und das Hapten Diphenylhydantoin sind, so ist der fluoreszenzmarkierte Hapten-Komplex hydrophob. Bei einer Reihe von üblichen Oberflächen auf Cellulose-Basis wird der fluoreszenzmarkierte Komplex nicht nur spezifisch am Antikörper, sondern unspezifisch an der gesamten festen Oberfläche gebunden.

Das Problem der niedrigen oder verminderten Antigenität des fluoreszenzmarkierten Hapten-Komplexes ist aller Wahrscheinlichkeit nach auf die sterische Hinderung zwischen dem Hapten und dem Fluoreszenzmolekülteil zurückzuführen. Beim fluoreszenzmarkierten Komplex vermindert die Nähe des hydrophoben Fluoreszenzmolekülteiles die Antikörper-Hapten-Annäherung. Außerdem bedingt dies die starke Bindung des Komplexes an Albumin oder andere Serumbestandteile. Die Folge hiervon ist ein vermindertes Fluoreszenzsignal, bekannt auch als »Fluoreszenzlöschung«.

Die genannten Probleme dürften auf die räumliche Nähe der Fluoreszenzverbindung und des Haptenmolekülteiles im Komplexmolekül zurückzuführen sein. Der hydrophobe Charakter der beiden Molekülteile verstärkt die Probleme.

Aufgabe der vorliegenden Erfindung ist es demzufolge, die nichtspezifische Bindung zwischen dem fluoreszenzmarkierten Hapten-Komplex und der festen Oberfläche stark zu vermindern. Eine weitere Aufgabe der vorliegenden Erfindung besteht in der Erhöhung der Antigenität des fluoreszenzmarkierten Haptens. Ferner ist es Aufgabe der vorliegenden Erfindung, die Fluoreszenzlöschung des fluoreszenzmarkierten Haptens durch Albumin oder andere Serumbestandteile zu vermindern.

Die Lösung der gestellten Aufgaben bestand überraschenderweise darin, daß zwischen die beiden oben genannten Molekülteile ein hydrophiles Zwischenglied eingebaut wird.

Zwar sind bereits Zwischenglieder beschrieben worden, doch dienten diese entweder nur dazu, die Hydrophilie des Komplexes zu verbessern: Einschub hydrophiler Verbindungen (EPA 0 003 583) oder dazu, die Nachweisgrenze der Methode zu erniedrigen, indem multifunktionelle Einheiten verwendet wurden, die eine Ansammlung fluoreszierender Verbindungen pro Komplex ermöglichen (US-P 4 169 137). Die durch die räumliche Nähe der Fluoreszenzverbindung und des Haptenmolekülteiles auftretenden oben genannten Probleme konnten diese Zwischenglieder nicht beseitigen; ganz im Gegenteil: die aus der US-P bekannten Zwischenglieder können zu einer noch stärkeren sterischen Hinderung führen.

Durch den Einbau der erfindungsgemäßen Zwischenglieder erzielt man ein verbessertes fluoreszierendes Reagenz und eine verbesserte Immunofluoreszenz-Bestimmungsmethode.

Gegenstand der Erfindung ist ein fluoreszierendes diagnostisches Reagenz, enthaltend eine Verbindung aus

a) einem hydrophoben Hapten,
b) einer hydrophilen Verbindung und
c) einer hydrophilen fluoreszierenden Verbindung auf Basis von Fluorescein, Umbelliferon oder Fluorescamin,

wobei sowohl das hydrophobe Hapten als auch die hydrophobe fluoreszierende Verbindung voneinander getrennt an die hydrophile Verbindung gebunden sind, in dem die hydrophile Verbindung ein Aminoglykosid der allgemeinen Formel

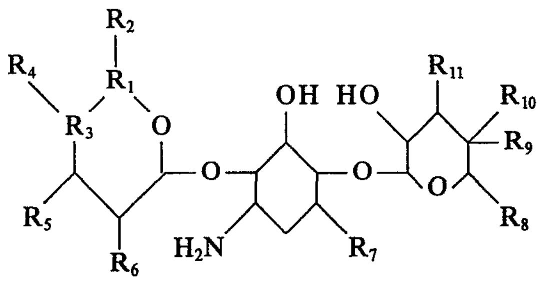

darstellt, in der bedeuten

$R_1$ =C oder —CH;

$R_2$ —$CH_2OH$, —$CH_2NH_2$, —$CH_2NHCH_3$,

$$-\underset{|}{\overset{CH_3}{|}}CHNH_2 \text{ oder } -\overset{CH_3}{\underset{}{|}}CHNHCH_3;$$

$R_3$ =C oder —CH;

$R_4$, $R_5$ und $R_9$: —H oder —OH;

$R_6$ —OH oder —$NH_2$;

$R_7$ —$NH_2$, —$NHCH_2CH_3$ oder —NH—$COCH(OH)CH_2CH_2NH_2$;

$R_8$ —H oder —$CH_2OH$;

$R_{10}$ —OH oder —$CH_3$ und

$R_{11}$ —$NH_2$ oder —$NHCH_3$.

Die Erfindung ist bezüglich des Haptens praktisch unbeschränkt anwendbar. Die einzige Voraussetzung besteht lediglich darin, daß das Hapten ein hydrophobes Molekül ist und entweder bereits eine funktionelle Molekülgruppe für die Bindung des hydrophilen Zwischengliedes besitzt oder durch Modifizierung mit einer derartigen Molekülgruppe ausgestattet werden kann. Hierbei handelt es sich um dieselben funktionellen Gruppen, die üblicherweise bei der Verknüpfung des Haptens mit der Fluoreszenzmarkierung verwendet werden, nämlich beispielsweise um Alkohol-, Äther-, Ester-, Amid-, Halogen-, Nitrilo- und Mercaptogruppen, speziell um Amino- und Carboxylgruppen. Es bestehen vielfältige Möglichkeiten, für das jeweils zu analysierende Hapten eine geeignete funktionelle Gruppe auszusuchen und diese entweder nachträglich oder bereits bei der Synthese des Haptens einzubauen. Im Falle der Haptene Phenobarbital, Diphenylhydantoin und Primidon haben sich beispielsweise die Aminogruppe und Carboxylgruppen wie Methylcarboxymethyl-, Methylcarboxybutyl-, Carboxymethyl- und Carboxybutylgruppen bewährt.

Die Erfindung ist insbesondere bei der Bestimmung von Antikonvulsiva wie beispielsweise Phenobarbital, Diphenylhydantoin und Primidon anwendbar. Diese Arzneimittel werden bei chronischen Krankheiten des Nervensystems, z. B. Epilepsie, verwendet. Da es bei einem Pharmakon in vielen Fällen von seiner Konzentration im Serum abhängt, ob es als Arzneimittel oder als Gift wirkt, ist seine genaue Konzentrationsbestimmung im Serum sehr wichtig. Mit Hilfe des erfindungsgemäßen Reagenzes und der erfindungsgemäßen Immunofluoreszenz-Bestimmung ist es möglich, diese Serumbestimmung durchzuführen.

Als hydrophiles Zwischenglied stehen aus der Klasse der genannten Aminoglykoside zahlreiche Verbindungen zur Verfügung. Diese hydrophilen Verbindungen haben wenigstens zwei reaktive Molekülstellen, welche sowohl das Hapten als auch die Fluoreszenzmarkierung zu binden vermögen.

Vorzugsweise werden als hydrophiles Zwischenglied ein Aminoglykosid-Antibioticum eingesetzt, insbesondere Gentamycine, Tobramycin, Amikacin, Sisomicin, Netilmicin und Kanamycine. Bevorzugte Aminoglykoside, die als hydrophiles Zwischenglied Verwendung finden können, sind in Tabelle I zusammengestellt.

Tabelle I

Aminoglykoside

| Nr. | Substituenten $R_1$ | $R_2$ | $R_3$ | $R_4$ | $R_5$ | $R_6$ | $R_7$ | $R_8$ | $R_9$ | $R_{10}$ | $R_{11}$ |
|---|---|---|---|---|---|---|---|---|---|---|---|
| 1. | $—CH$ | $—CH_2OH$ | $—CH$ | $—OH$ | $—OH$ | $—NH_2$ | $—NH_2$ | $—H$ | $—H$ | $—OH$ | $—NHCH_3$ |
| 2. | $—CH$ | $—CH_2NH_2$ | $—CH$ | $—H$ | $—H$ | $—NH_2$ | $—NH_2$ | $—H$ | $—OH$ | $—CH_3$ | $—NHCH_3$ |
| 3. | $—CH$ | $CH_3$<br>$—CHNHCH_3$ | $—CH$ | $—H$ | $—H$ | $—NH_2$ | $—NH_2$ | $—H$ | $—OH$ | $—CH_3$ | $—NHCH_3$ |
| 4. | $—CH$ | $CH_3$<br>$—CHNH_2$ | $—CH$ | $—H$ | $—H$ | $—NH_2$ | $—NH_2$ | $—H$ | $—OH$ | $—CH_3$ | $—NHCH_3$ |
| 5. | $—CH$ | $—CH_2NH_2$ | $—CH$ | $—OH$ | $—H$ | $—NH_2$ | $—NH_2$ | $—CH_2OH$ | $—H$ | $—OH$ | $—NH_2$ |
| 6. | $—CH$ | $—CH_2NH_2$ | $—CH$ | $—OH$ | $—OH$ | $—OH$ | $—NHCOCH(OH)$<br>$CH_2CH_2NH_2$ | $—CH_2OH$ | $—H$ | $—OH$ | $—NH_2$ |
| 7. | $=C$ | $—CH_2NH_2$ | $=C$ | $—H$ | $—H$ | $—NH_2$ | $—NH_2$ | $—H$ | $—OH$ | $—CH_3$ | $—NHCH_3$ |
| 8. | $=C$ | $—CH_2NH_2$ | $=C$ | $—H$ | $—OH$ | $—OH$ | $—NHCH_2CH_3$ | $—CH_2OH$ | $—H$ | $—OH$ | $—NH_2$ |
| 9. | $—CH$ | $—CH_2NH_2$ | $—CH$ | $—OH$ | $—OH$ | $—OH$ | $—NH_2$ | $—CH_2OH$ | $—H$ | $—OH$ | $—NH_2$ |
| 10. | $—CH$ | $—CH_2NH_2$ | $—CH$ | $—OH$ | $—OH$ | $—NH_2$ | $—NH_2$ | $—CH_2OH$ | $—H$ | $—OH$ | $—NH_2$ |
| 11. | $—CH$ | $—CH_2OH$ | $—CH$ | $—OH$ | $—OH$ | $—NH_2$ | $—NH_2$ | $—CH_2OH$ | $—H$ | $—OH$ | $—NH_2$ |
| 12. | $—CH$ | $—CH_2NHCH_3$ | $—CH$ | $—H$ | $—H$ | $—NH_2$ | $—NH_2$ | $—H$ | $—OH$ | $—CH_3$ | $—NHCH_3$ |

Die Bezeichnungen der in Tabelle I dargestellten Verbindungen werden in Tabelle II angegeben.

Tabelle II

Bezeichnung der in Tabelle I angegebenen Aminoglykoside.

| Nr. der Verbindungen in Tabelle I | Bezeichnung |
|---|---|
| 1 | Gentamicin A |
| 2 | Gentamicin*) $C_{1a}$ |
| 3 | Gentamicin*) $C_1$ |
| 4 | Gentamicin*) $C_2$ |
| 5 | Tobramicin |
| 6 | Amikacin |
| 7 | Sisomicin |
| 8 | Netilmicin |
| 9 | Kanamycin A |
| 10 | Kanamycin B |
| 11 | Kanamydin C |
| 12 | Sagamicin |

*) Gentamicin C ist ein Gemisch aus gleichen Teilen der unter Nr. 2 bis 4 genannten Isomeren.

Die hydrophobe fluoreszierende Verbindung kann aus der Vielzahl der bekannten fluoreszierenden Reagenzien ausgewählt werden, insbesondere aus den für die Fluoreszenzmarkierung üblichen Verbindungen. Beispiele geeigneter Verbindungen sind die Derivate des Fluoresceins, des Umbelliferons und des Fluorescamins. Besonders bevorzugt sind 5-Jodacetyl-aminofluorescein, 5-Aminofluorescein, Fluoresceinisothiocyanat, 5-Dichlortriazinyl-aminofluorescein und Rhodaminisothiocyanat. Fluoresceinisothiocyanat (R = $-NCS$) und 5-Aminofluorescein (R = $-NH_2$) besitzen die folgende Struktur:

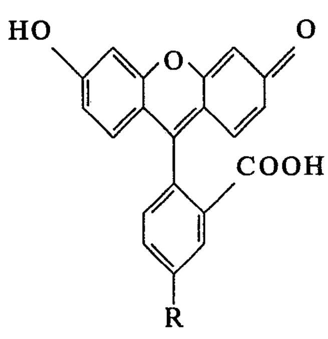

Vorzugsweise besitzt die hydrophobe fluoreszierende Verbindung nur eine reaktive Molekülstelle, da im Falle mehrerer reaktiver Molekülstellen die Tendenz besteht, daß die quantitative Messung des zu analysierenden Haptens weniger genau ist.

Das erfindungsgemäße fluoreszierende diagnostische Reagenz wird üblicherweise hergestellt, indem man zunächst das hydrophobe Hapten in der Form eines reaktiven Derivates mit der hydrophilen Verbindung umsetzt und dann das Reaktionsprodukt mit der fluoreszierenden Verbindung zur Reaktion bringt, um letztere an das hydrophile Zwischenglied zu binden.

Die Reaktionspartner werden im Molverhältnis von 1 : 1 : 1 zur Reaktion gebracht. Die Herstellung des Reagenzes erfolgt analog der bei der Synthese von fluoreszenzmarkierten Haptenen üblichen Weise und ergibt sich auch im einzelnen aus den Ausführungsbeispielen.

Bevorzugte erfindungsgemäße fluoreszierende Reagentien sind Verbindungen aus den oben genannten Aminoglykosiden der angegebenen allgemeinen Formel und 5-Aminofluorescein oder Fluoresceinisothiocyanat und einem Antikonvulsivum, insbesondere Diphenylhydantoin, Phenobarbital oder Primidon. Besonders bevorzugt sind die Verbindungen aus einem Antikonvulsivum, speziell Diphenylhydantoin, Fluoresceinisothiocyanat und Gentamicin sowie Verbindungen aus einem Antikonvulsivum, insbesondere Phenobarbital, Tobramycin und Fluoresceinisothiocyanat.

Ein weiterer Gegenstand der Erfindung ist eine Immunofluoreszenz-Bestimmungsmethode für Hapten-Antigene, die dadurch gekennzeichnet ist, daß man ein Reagenz verwendet, enthaltend eine Verbindung aus

a) einem hydrophoben Hapten,
b) einer hydrophilen Verbindung und
c) einer hydrophoben fluoreszierenden Verbindung auf Basis von Fluorescein, Ummbelliferon oder Fluorescamin,

wobei sowohl das Hapten als auch die hydrophobe fluoreszierende Verbindung voneinander getrennt an die hydrophile Verbindung gebunden sind. Die hydrophile Verbindung stellt ein Aminoglykosid der oben erwähnten allgemeinen Formel dar.

Bevorzugt wird die Immunofluoreszenz-Bestimmungsmethode, bei welcher die Verbindung aus einem Aminoglykosid der oben angegebenen allgemeinen Formel erhalten wurde. Nach weiteren bevorzugten Bestimmungsmethoden ist die im Reagenz enthaltene Verbindung aus dem obengenannten Aminoglykosid und 5-Aminofluorescein oder Fluoresceinisothiocyanat hergestellt, speziell aus einem Antikonvulsivum als Hapten, insbesondere aus Diphenylhydantoin, Phenobarbital oder Primidon. Nach einer besonders bevorzugten Bestimmungsmethode ist die im Reagenz enthaltene Verbindung aus einem Antikonvulsivum, speziell Diphenylhydantoin sowie Fluoresceinisothiocyanat und Gentamicin oder aus einem Antikonvulsivum als Hapten, insbesondere Phenobarbital, Tobramicin und Fluoresceinisothiocyanat synthetisiert worden.

## Beispiel

Ein fluoreszierendes diagnostisches Reagenz aus Diphenylhydantoin (DPH) als Hapten, Gentamicin als hydrophiler Verbindung und Fluoresceinisothiocyanat als fluoreszierende Verbindung wurde in folgender Weise hergestellt.

Herstellung des Diphenylhydantoin-Gentamicin-Derivates:

Zu der Lösung eines Carbonsäurederivates aus 0,5 mMol Diphenylhydantoin und 0,5 mMol N-Hydroxysuccinimid (NHS) in einem Milliliter wasserfreiem Dimethylformamid wurden 0,75 mMol Dicyclohexylcarbodiimid (DCC) hinzugefügt. Anschließend wurde das Gemisch noch eine Stunde lang bei Raumtemperatur gerührt und unter Rühren über Nacht im Kühlschrank gekühlt. Nach dem Abfiltrieren eines weißen Niederschlages wurde das Filtrat langsam zu der Lösung von 600 mg Gentamicin in 5 ml eines 0,01 molaren Carbonatpuffers mit einem pH von 9.0 zugefügt. Unter Gentamicin wird ein Gemisch aus den in Tabelle 1 unter den Nummern 2, 3 und 4 genannten Verbindungen verstanden. Der pH-Wert des Reaktionsgemisches wurde überwacht und konstant auf dem Wert 9.0 gehalten. Danach wurde das Reaktionsgemisch unter Rühren über Nacht im Kühlschrank gekühlt und anschließend bei 3000 Umdrehungen pro Minute 10 Minuten lang zentrifugiert. Der Überstand wurde unter Verwendung von Silicagel beschichteten Platten gereinigt, wobei dreimal mit einem Methanol-Chloroform-Gemisch im Verhältnis von 10 : 7 und einmal mit einem ammoniakalischen Methanol-Chloroform-Gemisch (Methanol : Chloroform : 5 n $NH_4OH$ = 2 : 1 :1) eluiert wurde. Die UV-positive Zone, welche erst beim Eluieren mit dem Methanol-Chloroform-5 n $NH_4OH$-Gemisch wanderte, wurde abgetrennt und mit einem Gemisch aus Methanol-Chloroform-5 n $NH_4OH$ extrahiert. Nach dem Entfernen des Lösungsmittels unter vermindertem Druck wurde das Diphenylhydantoin-Gentamicin als Rückstand erhalten.

Herstellung des Diphenylhydantoin-Gentamicin-Fluoresceinisothiocyanat-Derivates:

Die Synthese erfolgt analog der Herstellung des Diphenylhydantoin-Gentamicin-Derivates.

Das in der oben beschriebenen Weise erhaltene Diphenylhydantoin-Gentamicin-Derivat wurde in 2 ml eines 0,01 molaren Carbonatpuffers (pH 9.0) gelöst und portionsweise mit einer äquimolaren Menge Fluoresceinisothiocyanat versetzt. Danach wurde das Reaktionsgemisch unter Ausschluß von Licht bei Raumtemperatur über Nacht gerührt. Danach war die Reaktion beendet. Der Niederschlag wurde durch Zentrifugieren abgetrennt. Der Überstand wurde unter Verwendung Silicagel beschichteter Platten gereinigt, wobei das Eluieren in der oben beschriebenen Weise erfolgte.

Die fluoreszierende Zone wurde abgetrennt und mit einem Gemisch aus Methanol : Chloroform : 5 n $NH_4OH$ extrahiert. Das Lösungsmittel wurde unter vermindertem Druck entfernt und der feste Rückstand in destilliertem Wasser, dem ein Tropfen konzentriertes Ammoniak zugesetzt worden war, aufgenommen.

In der Tabelle III werden die Wasserlöslichkeiten der Haptene Phenobarbital und Diphenylhydantoin in unveränderter Form, in modifizierter Form (carboxyliert bzw. aminiert) und in der sogenannten konjugierten Form, nämlich als Umsetzungsprodukt mit Gentamicin gegenübergestellt. Hieraus ergibt sich, daß die Haptene in unveränderter und in modifizierter Form hydrophob sind (Die Unlöslichkeit in Wasser ist ein Maß für die Hydrophobie einer Verbindung). Mit steigender Unlöslichkeit der Verbindungen in Wasser steigt die Hydrophobie und die Wahrscheinlichkeit, daß sie an die Oberfläche gebunden werden, wobei durch die unspezifischen Bindungen große Ungenauigkeiten in der Immunofluoreszenz-Bestimmungsmethode verursacht werden. Wie aus der Tabelle III weiterhin ersichtlich, erhält man ein lösliches, d. h. hydrophiles Produkt, wenn man ein hydrophobes Hapten wie Phenobarbital oder Diphenylhydantion mit einer hydrophilen Verbindung wie Gentamicin verbindet. Derartige Produkte werden nicht als solche an die feste Oberfläche gebunden.

Tabelle III

Vergleich der Wasserlöslichkeiten

| | Unmodifiziertes Hapten | Modifiziertes Hapten Carboxyliert*) | Modifiziertes Hapten Aminiert | Carboxyliertes und mit Gentamicin umges. Produkt |
|---|---|---|---|---|
| Phenobarbital | unlöslich | unlöslich | unlöslich | löslich |
| Diphenylhydantoin | unlöslich | unlöslich | unlöslich | löslich |

*) Die carboxylierten Verbindungen sind entweder ein Methylcarboxymethyl-, ein Methylcarboxybutyl-, ein Carboxymethyl- oder ein Carboxybutylderivat.

Da die üblichen Fluoreszenzreagenzien hydrophob sind, lagern sich Albumin und andere Serumproteine an sie an und verhindern oder vermindern deren Fähigkeit zur Fluoreszenz, wenn sie bei der Immunofluoreszenz-Bestimmung dem Anregungsstrahl ausgesetzt werden. In der Tabelle IV wird der fluoreszenzlöschende Effekt von Albumin am Beispiel zweier Fluoreszenz-Reagenzien, nämlich des Fluoresceinisothiocyanats und des 5-Aminofluoresceins dargestellt, und zwar am unveränderten Fluoreszenz-Reagenz, am Umsetzungsprodukt mit dem Hapten sowie am erfindungsgemäßen, aus Hapten fluoreszierender Verbindung und einer hydrophilen Verbindung erhaltenem Reagenz (Hapten-Gentamicin-Konjugat).

Tabelle IV

Fluoreszenzlöschung durch Albumin

| Fluoreszierende Verbindung | unveränderte fluoreszierende Verbindung | Umsetzungsprodukt mit dem Hapten | Hapten-Gentamicin-Konjugat |
|---|---|---|---|
| Fluorescein-isothiocyanat | ja | ja | nein |
| Aminofluorescein | ja | ja | nein |

Die nachstehende Tabelle V belegt die verbesserte Antigenität des Haptens, welches einen Bestandteil des erfindungsgemäßen fluoreszierenden Reagenzes bildet. Wenn man beispielsweise das Hapten Diphenylhydantoin nur mit der hydrophoben fluoreszierenden Verbindung kombiniert, so ist seine Antigenität, das ist die Fähigkeit, sich mit dem korrespondierenden Antikörper zu verbinden, beträchtlich vermindert; wenn jedoch erfindungsgemäß zusätzlich eine hydrophile Verbindung wie z. B. das Gentamicin eingebaut wird, ist die Antigenität höher als bei dem Reaktionsprodukt aus Hapten und der fluoreszierenden Verbindung oder ist ebenso hoch wie die des reinen Haptens.

Tabelle V

Antigenität von Diphenylhydantoin (DPH) bezogen auf DPH in %

| Unmodifiziertes DPH | Reaktionsprodukt des DPH mit einer fluoreszierenden Verbindung | | Reaktionsprodukt des DPH mit Gentamicin und einer fluoreszierenden Verbindung | | | |
|---|---|---|---|---|---|---|
| | Carboxymethyl-diphenyl-hydantoin-amino-fluorescein | N-Amino-propyl-diphenyl-hydantoin-fluorescein-isothio-cyanat | Carboxy-propyl-diphenyl-hydantoin-gentamicin-fluorescein-isothio-cyanat*) | Carboxy-propyl-diphenyl-hydantoin-gentamicin-fluorescein-isothio-cyanat*) | Carboxy-propyl-diphenyl-hydantoin-gentamicin-fluorescein-isothio-cyanat*) | Carboxy-propyl-diphenyl-hydantoin-gentamicin-fluorescein-isothio-cyanat |
| 100 | 17 | 30 | 100 | 101 | 58 | 150 |

*) Es wurden drei verschiedene Verbindungen abgetrennt, sie entsprechen den in Tabelle I aufgeführten Gentamicin-Isomeren.

Aus dem Ausführungsbeispiel wird ersichtlich, daß der Einbau einer hydrophilen Verbindung alle Schwierigkeiten löst, welche bei der Immunofluoreszenz-Bestimmungsmethode auftreten, wenn ein hydrophobes Testmolekül, wie z. B. ein Hapten mit einer hydrophoben Markierung, wie z. B. einer fluoreszierenden Verbindung kombiniert wird. Damit ist nachgewiesen, daß die erfindungsgemäßen fluoreszierenden Reagenzien die Nachteile der bislang üblichen Fluoreszenz-Bestimmungsmethoden beseitigt haben.

**Patenansprüche**

1. Fluoreszierendes diagnostisches Reagenz, enthaltend eine Verbindung aus

a) einem hydrophoben Hapten,
b) einer hydrophilen Verbindung und
c) einer hydropholen fluoreszierenden Verbindung auf Basis von Fluorescein, Umbelliferon oder Fluorescamin,

wobei sowohl das hydrophobe Hapten als auch die hydrophobe fluoreszierende Verbindung voneinander getrennt an die hydrophile Verbindung gebunden sind, dadurch gekennzeichnet, daß die hydrophile Verbindung ein Aminoglykosid der allgemeinen Formel

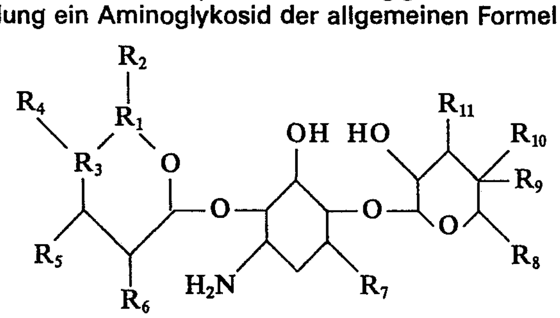

ist, in welcher bedeuten:

$R_1$ $=C$ oder $-CH$;

$R_2$ $-CH_2OH$, $-CH_2NH_2$, $-CH_2NHCH_3$,

$-\overset{CH_3}{\overset{|}{C}}HNH_2$ oder $-\overset{CH_3}{\overset{|}{C}}HNHCH_3$;

$R_3$ $=C$ oder $-CH$;

$R_4$, $R_5$ und $R_9$: $-H$ oder $-OH$ ;

$R_6$ $-OH$ oder $-NH_2$;

$R_7$ $-NH_2$, $-NHCH_2CH_3$ oder $-NH-COCH(OH)CH_2CH_2NH_2$;

$R_8$ $-H$ oder $-CH_2OH$;

$R_{10}$ $-OH$ oder $-CH_3$ und

$R_{11}$ $-NH_2$ oder $-NHCH_3$.

2. Fluoreszierendes diagnostisches Reagenz nach Anspruch 1, dadurch gekennzeichnet, daß die hydrophobe fluoreszierende Verbindung 5-Aminofluorescein ist.

3. Fluoreszierendes diagnostisches Reagenz nach Anspruch 1, dadurch gekennzeichnet, daß die hydrophobe fluoreszierende Verbindung Fluoresceinisothiocyanat ist.

4. Fluoreszierendes diagnostisches Reagenz nach den Ansprüchen 2 und 3, dadurch gekennzeichnet, daß das Hapten ein Antikonvulsivum ist.

5. Fluoreszierendes diagnostisches Reagenz nach Anspruch 4, dadurch gekennzeichnet, daß das Antikonvulsivum Diphenylhydantoin ist.

6. Fluoreszierendes diagnostisches Reagenz nach den Ansprüchen 4 und 5, dadurch gekennzeichnet, daß die hydrophobe fluoreszierende Verbindung Fluoresceinisothiocyanat und das Aminoglykosid Gentamicin ist.

7. Fluoreszierendes diagnostisches Reagenz nach Anspruch 4, dadurch gekennzeichnet, daß das Antikonvulsivum Phenobarbital ist.

8. Fluoreszierendes diagnostisches Reagenz nach Anspruch 7, dadurch gekennzeichnet, daß die fluoreszierende Verbindung Fluoresceinisothiocyanat und das Aminoglykosid Tobramycin sind.

9. Fluoreszierendes diagnostisches Reagenz nach Anspruch 4, dadurch gekennzeichnet, daß das Antikonvulsivum Primidon ist.

10. Immunofluoreszenz-Bestimmungsmethode für Hapten-Antigene, dadurch gekennzeichnet, daß man ein Reagenz verwendet, enthaltend eine Verbindung aus

a) einem hydrophoben Hapten,
b) einer hydrophilen Verbindung und
c) einer hydrophoben fluoreszierenden Verbindung auf Basis von Fluorescein, Umbelliferon oder Fluorescamin,

wobei sowohl das hydrophobe Hapten als auch die hydrophobe fluoreszierende Verbindung voneinander getrennt an die hydrophile Verbindung gebunden sind, dadurch gekennzeichnet, daß die hydrophile Verbindung ein Aminoglykosid der allgemeinen Formel

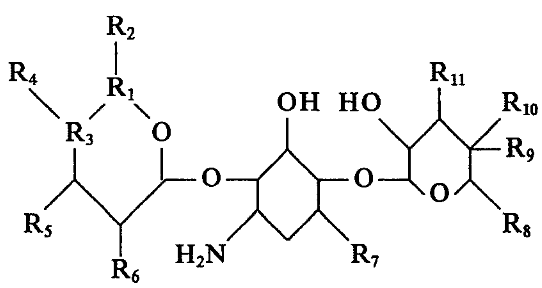

ist, in welcher bedeuten:

$R_1$ $=C$ oder $-CH$;

$R_2$ $-CH_2OH$, $-CH_2NH_2$, $-CH_2NHCH_3$,

$-CH(CH_3)NH_2$ oder $-CH(CH_3)-NH-CH_3$;

$R_3$ $=C$ oder $-CH$;

$R_4$, $R_5$ und $R_9$: $-H$ oder $-OH$;

$R_6$ $-OH$ oder $-NH_2$;

$R_7$ $-NH_2$, $-NHCH_2CH_3$ oder $-NHCOCH(OH)CH_2-CH_2-NH_2$;

$R_8$ —H oder —$CH_2OH$;

$R_{10}$ —OH oder —$CH_3$ und

$R_{11}$ —$NH_2$ oder —$NHCH_3$.

11. Immunofluoreszenz-Bestimmungsmethode nach Anspruch 10, dadurch gekennzeichnet, daß die hydrophobe fluoreszierende Verbindung 5-Aminofluorescein ist.

12. Immunofluoreszenz-Bestimmungsmethode nach Anspruch 10, dadurch gekennzeichnet, daß die hydrophobe fluoreszierende Verbindung Fluoresceinisothiocyanat ist.

13. Immunofluoreszenz-Bestimmungsmethode nach den Ansprüchen 11 und 12, dadurch gekennzeichnet, daß das Hapten ein Antikonvulsivum ist.

14. Immunofluoreszenz-Bestimmungsmethode nach Anspruch 13, dadurch gekennzeichnet, daß Antikonvulsivum Diphenylhydantoin ist.

15. Immunofluoreszenz-Bestimmungsmethode nach Anspruch 14, dadurch gekennzeichnet, daß die hydrophile fluoreszierende Verbindung Fluoresceinisothiocyanat und das Aminoglykosid Gentamicin ist.

16. Immunofluoreszenz-Bestimmungsmethode nach Anspruch 13, dadurch gekennzeichnet, daß das Antikonvulsivum Phenobarbital ist.

17. Immunofluoreszenz-Bestimmungsmethode nach Anspruch 16, dadurch gekennzeichnet, daß die hydrophile fluoreszierende Verbindung Fluoresceinisothiocyanat und das Aminoglykosid Tobramycin sind.

18. Immunofluoreszenz-Bestimmungsmethode nach Anspruch 13, dadurch gekennzeichnet, daß das Antikonvulsivum Primidon ist.

## Claims

1. Fluorescent diagnostic reagent, containing a compound consisting of

a) a hydrophobic hapten,
b) a hydrophilic compound and
c) a hydrophobic fluorescent compound based on fluoresceine, umbelliferone or fluorescamine,

whilst both the hydrophobic hapten and also the hydrophobic fluorescent compound are bonded to the hydrophilic compound separately from each other, characterised in that the hydrophilic compound is an aminoglycoside of general formula

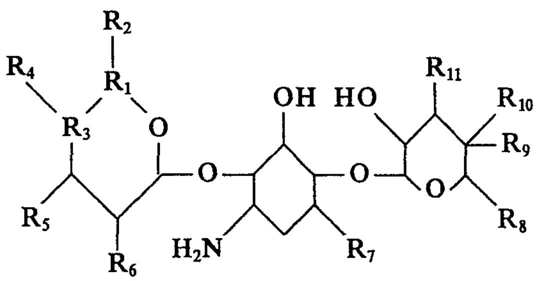

wherein:

$R_1$ represents =C or —CH;

$R_2$ represents —$CH_2OH$, —$CH_2NH_2$, —$CH_2NHCH_3$,

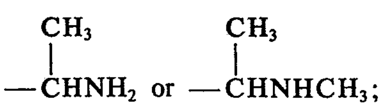

$R_3$ represents =C or —CH;

$R_4$, $R_5$ and $R_9$ represent —H or —OH;

$R_6$ represents —OH or —$NH_2$;

$R_7$ represents —$NH_2$, —$NHCH_2CH_3$ or —NH—$COCH(OH)CH_2CH_2NH_2$;

$R_8$ represents —H or —$CH_2OH$;

$R_{10}$ represents —OH or —$CH_3$ and

$R_{11}$ represents —$NH_2$ or —$NHCH_3$.

2. Fluorescent diagnostic reagent as claimed in claim 1, characterised in that the hydrophobic fluorescent compound is 5-aminofluoresceine.

3. Fluorescent diagnostic reagent as claimed in claim 1, characterised in that the hydrophobic fluorescent compound is fluoresceineisothiocyanate.

4. Fluorescent diagnostic reagent as claimed in claims 2 and 3, characterised in that the hapten is an anticonvulsive.

5. Fluorescent diagnostic reagent as claimed in claim 4, characterised in that the anticonvulsive is diphenylhydantoin.

6. Fluorescent diagnostic reagent as claimed in claims 4 and 5, characterised in that the hydrophobic fluorescent compound is fluoresceineisothiocyanate and the aminoglycoside is gentamicine.

7. Fluorescent diagnostic reagent as claimed in claim 4, characterised in that the anticonvulsive is phenobarbital.

8. Fluorescent diagnostic reagent as claimed in claim 7, characterised in that the fluorescent compound is fluoresceineisothiocyanate and the aminoglycoside is tobramycin.

9. Fluorescent diagnostic reagent as claimed in claim 4, characterised in that the anticonvulsive is primidone.

10. Immunofluorescent method of determining hapten antigens, characterised in that a reagent is used containing a compound consisting of

a) a hydrophobic hapten,
b) a hydrophilic compound and
c) a hydrophobic fluorescent compound based on fluoresceine, umbelliferone or fluorescamine,

in which both the hydrophobic hapten and also the hydrophobic fluorescent compound are bonded to the hydrophilic compound separately from each other, characterised in that the hydrophilic compound is an aminoglycoside of general formula

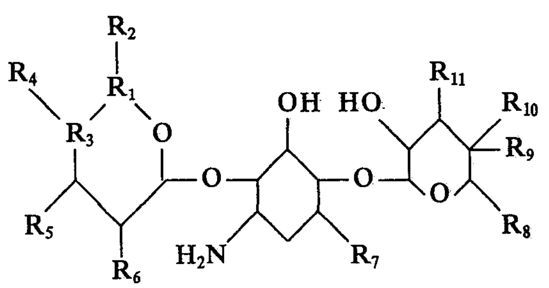

wherein:

$R_1$ represents $=C$ or $-CH$;

$R_2$ represents $-CH_2OH$, $-CH_2NH_2$, $-CH_2NHCH_3$,

$$-\underset{|}{\overset{CH_3}{}}CHNH_2 \text{ or } -\overset{CH_3}{\underset{}{|}}CHNHCH_3;$$

$R_3$ represents $=C$ or $-CH$;

$R_4$, $R_5$ and $R_9$ represent $-H$ or $-OH$;

$R_6$ represents $-OH$ or $-NH_2$;

$R_7$ represents $-NH_2$, $-NHCH_2CH_3$ or $-NH-COCH(OH)CH_2CH_2NH_2$;

$R_8$ represents $-H$ or $-CH_2OH$;

$R_{10}$ represents $-OH$ or $-CH_3$ and

$R_{11}$ represents $-NH_2$ or $-NHCH_3$.

11. Immunofluorescent method of determination as claimed in claim 10, characterised in that the hydrophobic fluorescent compound is 5-aminofluoresceine.

12. Immunofluorescent method of determination as claimed in claim 10, characterised in that the hydrophobic fluorescent compound is fluoresceineisothiocyanate.

13. Immunofluorescent method of determination as claimed in claims 11 and 12, characterised in that the hapten is an anticonvulsive.

14. Immunofluorescent method of determination as claimed in claim 13, characterised in that the anticonvulsive is diphenylhydantoin.

15. Immunofluorescent method of determination as claimed in claim 14, characterised in that the hydrophilic fluorescent compound is fluoresceineisothiocyanate and the aminoglycoside is gentamicine.

16. Immunofluorescent method of determination as claimed in claim 13, characterised in that the anticonvulsive is phenobarbital.

17. Immunofluorescent method of determination as claimed in claim 16, characterised in that the hydrophilic fluorescent compound is fluoresceineisothiocyanate and the aminoglycoside is tobramycin.

18. Immunofluorescent method of determination as claimed in claim 13, characterised in that the anticonvulsive is primidone.

**Revendications**

1. Réactif diagnostique fluorescent contenant un composé

a) d'un haptène hydrophobe
b) d'un composé hydrophile et
c) d'un composé fluorescent hydrophobe à base de fluorescéine, d'umbelliférone ou de fluorescamine,

où tant le haptène hydrophobe qu'également le composé fluorescent hydrophobe sont liés, séparés l'un de l'autre, au composé hydrophile, caractérisé en ce que le composé hydrophile est un aminoglycoside de formule générale

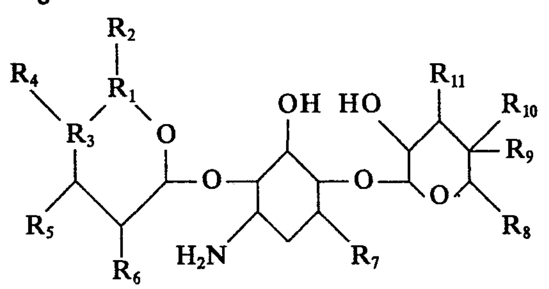

dans laquelle:

$R_1$ représente $=C$ ou $-CH$;

$R_2$ représente $-CH_2OH$, $-CH_2NH_2$, $-CH_2NHCH_3$,

$-CH(CH_3)NH_2$ ou $-CH(CH_3)NHCH_3$;

$R_3$ représente $=C$ ou $-CH$;

$R_4$, $R_5$ et $R_9$ représentent $-H$ ou $-OH$;

$R_6$ représente $-OH$ ou $-NH_2$;

$R_7$ représente $-NH_2$, $-NHCH_2CH_3$ ou $-NH-COCH(OH)CH_2CH_2NH_2$;

$R_8$ représente $-H$ ou $-CH_2OH$;

$R_{10}$ représente $-OH$ ou $-CH_3$ et

$R_{11}$ représente $-NH_2$ ou $-NHCH_3$.

2. Réactif diagnostique fluorescent selon la revendication 1, caractérisé en ce que le composé fluorescent hydrophobe est la 5-aminofluorescéine.

3. Réactif diagnostique fluorescent selon la revendication 1, caractérisé en ce que le composé fluorescent hydrophobe est l'isothiocyanate de fluorescéine.

4. Réactif diagnostique fluorescent selon les revendications 2 et 3, caractérisé en ce que le haptène est un agent anticonvulsif.

5. Réactif diagnostique fluorescent selon la revendication 4, caractérisé en ce que l'agent anticonvulsif est la diphénylhydantoine.

6. Réactif diagnostique fluorescent selon les revendications 4 et 5, caractérisé en ce que le composé fluorescent hydrophobe est l'isothiocyanate de fluorescéine et l'aminoglycoside est de la gentamycine.

7. Réactif diagnostique fluorescent selon la revendication 4, caractérisé en ce que l'agent anticonvulsif est le phénobarbital.

8. Réactif diagnostique fluorescent selon la revendication 7, caractérisé en ce que le composé fluorescent est l'isothiocyanate de fluorescéine et l'aminoglycoside est la tobramycine.

9. Réactif diagnostique fluorescent selon la revendication 4, caractérisé en ce que l'agent anticonvulsif est la primidone.

10. Procédé immunofluorescent de détermination pour des haptène-antigènes, caractérisé en ce qu'on utilise un réactif contenant un composé:

a) d'un haptène hydrophobe,
b) d'un composé hydrophile et
c) d'un composé fluorescent hydrophobe à base de fluorescéine, d'umbelliférone ou de fluorescamine,

où tant le haptène hydrophobe qu'également le composé fluorescent hydrophobe sont liés, séparés l'un de l'autre, au composé hydrophile, caractérisé en ce que le composé hydrophile est un aminoglycoside de formule générale

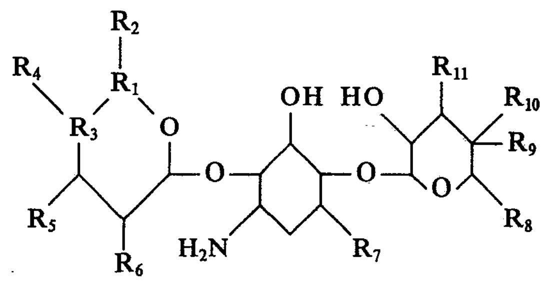

dans laquelle:

$R_1$ représente $=C$ ou $-CH$;

$R_2$ représente $-CH_2OH$, $-CH_2NH_2$, $-CH_2NHCH_3$,

$$-\overset{CH_3}{\overset{|}{C}}HNH_2 \text{ ou } -\overset{CH_3}{\overset{|}{C}}HNHCH_3;$$

$R_3$ représente $=C$ ou $-CH$;

$R_4$, $R_5$ et $R_9$ représentent $-H$ ou $-OH$;

$R_6$ représente $-OH$ ou $-NH_2$;

$R_7$ représente $-NH_2$, $-NHCH_2CH_3$ ou $-NH-COCH(OH)CH_2CH_2NH_2$;

$R_8$ représente $-H$ ou $-CH_2OH$;

$R_{10}$ représente $-OH$ ou $-CH_3$ et

$R_{11}$ représente $-NH_2$ ou $-NHCH_3$.

11. Procédé immunofluorescent de détermination selon la revendication 10, caractérisé en ce que le composé fluorescent hydrophobe est la 5-aminofluorescéine.

12. Procédé immunofluorescent de détermination selon la revendication 10, caractérisé en ce que le composé fluorescent hydrophobe est l'isothiocyanate de fluorescéine.

13. Procédé immunofluorescent de détermination selon les revendications 11 et 12, caractérisé en ce que le haptène est un agent anticonvulsif.

14. Procédé immunofluorescent de détermination selon la revendication 13, caractérisé en ce que l'agent anticonvulsif est la diphénylhydantoine.

15. Procédé immunofluorescent de détermination selon la revendication 14, caractérisé en ce que le composé fluorescent hydrophile est l'isothiocyanate de fluorescéine et l'aminoglycoside est la gentamycine.

16. Procédé immunofluorescent de détermination selon la revendication 13, caractérisé en ce que l'agent anticonvulsif est le phénobarbital.

17. Procédé immunofluorescent de détermination selon la revendication 16, caractérisé en ce que le composé fluorescent hydrophile est l'isothiocyanate de fluorescéine et l'aminoglycoside est la tobramycine.

18. Procédé immunofluorescent de détermination selon la revendication 13, caractérisé en ce que l'agent anticonvulsif est la primidone.